# EUROPEAN PATENT APPLICATION

(11) **EP 2 047 851 A1**
(43) Date of publication of application: **15.04.2009**
(21) Application number: 06782392.2
(22) Date of filing: 04.08.2006
(51) Int. Cl.: A61K 31/47, A61K 31/216, A61P 1/16, A61P 1/18, A61P 3/00, A61P 3/06, A61P 3/08, A61P 3/10, A61P 7/02

(54) **DRUG FORMULATION CONTAINING FIBRATE MEDICAMENT AND PROCESS FOR PRODUCING THE SAME**

(71) Applicant: ASKA Pharmaceutical Co., Ltd., Minato-ku, Tokyo 108-8532 (JP)
(72) Inventor: KANAZAWA, Hashime, Hamura-shi, Tokyo 205-8501 (JP); MORIMOTO, Masaya, Hamura-shi, Tokyo 205-8501 (JP); TANIMORI, Naoto, Hamura-shi, Tokyo 205-8501 (JP)
(74) Representative: Helbing, Jörg
(86) International application number: PCT/JP2006/315534
(87) International publication number: WO 2008/015763

(57) **Abstract**

A preparation (pharmaceutical composition) reducing a concentration of a free fatty acid and/or fibrinogen in the blood is prepared. The preparation contains a statin agent comprising at least a statin compound having a benzopyridine skeleton (e.g., pitavastatin) and a fibrate agent (e.g., fenofibrate). The preparation is useful as a preventive or treating agent for hyper-free fatty acidemia, metabolic syndrome, or type II diabetes.

## Description

### TECHNICAL FIELD

The present invention relates to a preparation which contains a statin agent and a fibrate agent and is useful for a preventive (or prophylactic) or treating (or therapeutic) agent for metabolic syndrome, type II diabetes (non-insulin-dependent diabetes mellitus), type II diabetes complication, or other diseases, and to a process for producing the preparation.

### BACKGROUND ART

Statin agents (or statin drugs) have an action to suppress synthesis of cholesterol by inhibiting hydroxymethylglutaryl-CoA (HMG-CoA) reductase, which is a rate-limiting enzyme in cholesterol biosynthesis pathway, and have been widely used as a preventive (or prophylactic) or treating (or therapeutic) agent for hyperlipidemia. Pravastatin, simvastatin, fluvastatin, atorvastatin, lovastatin, cerivastatin, rosuvastatin, and others have been known as representative statin agents. These statin agents have relatively highly effective in decreasing concentrations of total cholesterol and low-density lipoprotein (LDL)-binding cholesterol in the blood. However, these statin agents are not much effective in decreasing a triglyceride concentration in the blood, decreasing a free fatty acid concentration in the blood, and increasing a high-density lipoprotein (HDL)-binding cholesterol.

Accordingly, a preparation (or agent) which can reduce the total cholesterol concentration, the LDL-cholesterol concentration, the triglyceride concentration, and the free fatty acid concentration and increase the HDL-cholesterol concentration has been required. In particular, a preparation (pharmaceutical composition) which shows the above-mentioned pharmacological activities in a low dose has been desired.

Fibrate agents (or fibrate drugs) have an action to not only reduce a LDL cholesterol and a triglyceride by inhibiting synthesis or secretion of the triglyceride in the liver but also increase an HDL cholesterol and have been known as an agent for preventing or treating hyperlipidemia. However, the fibrate agent does not have a very high activity in decreasing the total cholesterol in the blood.

Incidentally, the followings have been known: each of the statin agent and the fibrate agent is used not only alone but also in combination with various agents. For example, the following combinations have been known: (1) a pharmaceutical composition containing a combination of fenofibrate and bezafibrate with metformin known as a remedy for diabetes, for reducing hyperglycemia due to non-insulin-dependent diabetes (e.g., see Patent Document 1), (2) a therapeutic agent for hyperlipidemia, atherosclerosis, or hypercholesterolemia, which comprises fenofibrate, bezafibrate, or clinofibrate, in combination with a cholesteryl ester transfer protein inhibiting compound (e.g., see Patent Document 2), (3) a therapeutic agent for hyperlipidemia, atherosclerosis, or hypercholesterolemia, which comprises fenofibrate, bezafibrate, or clinofibrate, in combination with an ileal bile acid transport inhibiting compound (e.g., see Patent Document 3), (4) an agent for the prophylaxis or treatment of atherosclerosis, hypercholesterolemia, and hyperlipoproteinemia, which comprises lovastatin or cerivastatin, in combination with a β-blocked (e.g., see Patent Document 4), (5) a TNF-α, inhibitor useful as an agent for the prophylaxis or treatment of inflammatory diseases, which comprises pravastatin or cerivastatin, in combination with pioglitazone known as an insulin sensitizer (e.g., see Patent Document 5), (6) a therapeutic agent for angina, atherosclerosis, or combined hyperlipidemia and hypertension, which comprises pravastatin or simvastatin, in combination with amlodipine as a therapeutic agent for hypertension (e.g., see Patent Document 6), (7) an action to reduce a triglyceride level in the blood by a combination of cerivastatin and fenofibrate (e.g., see Patent Document 7), and (8) an action to reduce a triglyceride level in the blood by a combination of atorvastatin and fenofibrate (e.g., see Non-patent Document 1).

Moreover, WO02/67901 (Patent Document 8) discloses a dosage form of a pharmaceutical composition containing a combination of a statin and a micronized fenofibrate which is stabilized by a phospholipid surface active substance for substantially increasing the bioavailability of the fibrate and substantially reducing the difference between the amount of the active species of the drug absorbed in a patient in the fasting state and the amount of the active species of the drug absorbed in the patient in the fed state. This document also discloses that the dosage form provides a therapeutically effective dose of the statin and a therapeutically effective quantity of the fenofibrate active species for a patient in need of treatment with the statin and fenofibrate, and the quantity of the fenofibrate active species given to the patient in the fasting state is at least 80% of the quantity of the fenofibrate active species given to the patient to whom a fat-containing meal is fed. WO03/26573 (Patent Document 9) discloses a method for appropriately selecting a statin and a non-statin agent for co-administration to a patient in need of such a co-administered agent treatment. WO2005/034908 (Patent Document 10) discloses a particulate material comprising as active substances fibrate(s) and statin(s) or a pharmaceutically active salt thereof, wherein at least 80% w/w of the total amount of active substances is dissolved in vehicle selected from the group consisting of a hydrophobic, a hydrophilic and a water-miscible vehicles and mentions that the material can improve the bioavailability of the fibrate and/or statin. In "Medical Consultation & New Remedies" vol. 40, No. 9 (September, 2003), pp. 779 to 785 (Non-patent Document 2), there is a description that the observation of the presence of drug interactions by comparison in pharmacokinetic parameter of pitavastatin in a dose of 4 mg administered a day for 6 days with pitavastatin administered in combination with fenofibrate in a dose of 160 mg administered a day or gemfibrozil in a dose of 600 mg administered twice a day for 7 days reveals a low degree of the drug interactions because of a low possibility of excessive increase in the concentration of unchanged pitavastatin in the blood even in the case of the combination use of these drugs.

However, these documents do not specifically disclose a preparation(e.g.,a pharmaceutical preparation) comprising a statin compound having benzopyridine skeleton (e.g., pitavastatin) as a HMG-CoA reductase inhibitor in combination with a fibrate agent.

Further, in association with obesity or diabetes, metabolic syndrome caused by visceral fat accumulation (visceral fat obesity) has been attracted the attention in recent years. The metabolic syndrome means a disease group concurrently having a plurality factors (arteriosclerosis risk factors) such as obesity, abnormal glucose tolerance, hypertension, and abnormal lipid metabolism. There were many unknown points about expression mechanisms of such factors. However, it is being known that obesity, particularly, visceral fat obesity, is closely related to a development of metabolic syndrome.

For example, in "Therapeutics", vol. 39, No. 6 (2005), pp. 59 to 62 (Non-patent Document 3) and "Adiposcience", vol. 3, No. 1 (2006), pp. 64 to 70 (Non-patent Document 4), a relationship between metabolic syndrome and increase of free fatty acid in the blood is reported. According to the reports, an excessive increase of a free fatty acid which is fed from visceral fat induces abnormal lipid metabolism. Specifically, a free fatty acid sustainedly (or continuously) fed from visceral fat or a free fatty acid retained in the blood causes hyper-free fatty acidemia (a disease which raises a hyper concentration of a free fatty acid in the blood). Such a free fatty acid flows into a non-adipose tissue such as liver, muscle, pancreatic β-cell and is excessively taken up therein, whereby various functional disorders of cells such as an inhibition of an insulin action (an inhibition of insulin-dependent glucose absorption (lipotoxicity)) are generated. Incidentally, in type II diabetes, a free fatty acid is secreted even when the diabetes is not accompanied by obesity, whereby a disorder of an insulin action is caused. Thus visceral fat obesity, which is a cause of an excessive free fatty acid, is a maj or risk factor of metabolic syndrome, and the free fatty acid particularly induces abnormal glycolipid metabolism as lipotoxicity.

On the other hand, WO2006/011495 (Patent Document 11) discloses a preparation comprising pitavastatin and fenofibrate in combination as an agent for treating hypercholesterolemia and/or hypertriglyceridemia. However, this document does not refer to a mixture ratio of pitavastatin and fenofibrate. Further, in Examples a combination effect of these active components are not enough, and the document fails to disclose or suggest an action to decrease a free fatty acid and fibrinogen, which is a risk factor independent of the decrease of cholesterol or triglyceride.

Further, the Patent Document does not refer to statement of metabolic syndrome or a disease derived therefrom, particularly a relationship between such a disease and a free fatty acid, at all. Furthermore, metabolic syndrome caused by such a visceral fat obesity is a risk factor of arteriosclerosis and also has a close relation to thrombus formation.
[Patent Document 1] WO99/40904
[Patent Document 2] WO00/38724
[Patent Document 3] WO00/38727
[Patent Document 4] WO01/74394
[Patent Document 5] US2003/60488
[Patent Document 6] WO03/26573
[Patent Document 7] US6511985
[Patent Document 8] WO02/67901 (claim 1)
[Patent Document 9] WO03/26573 (claim 1)
[Patent Document 10] WO2005/034908 (claim 1)
[Patent Document 11] WO2006/011495 (claim 1, paragraph number [0026], Example 1)
[Non-patent Document 1] "Diabetes Care", vol. 25 (2002), pp. 1198 to 1202)
[Non-patent Document 2] "Medical Consultation & New Remedies", vol. 40, No. 9 (September 2003), pp. 779 to 785
[Non-patent Document 3] "Therapeutics", vol. 39, No. 6 (2005), pp. 59 to 62
[Non-patent Document 4] "Adiposcience", vol. 3, No. 1 (2006), pp. 64 to 70

### DISCLOSURE OF THE INVENTION

### PROBLEMS TO BE SOLVED BY THE INVENTION

It is therefore an object of the present invention to provide a preparation which can drastically reduce a concentration of a free fatty acid in the blood.

Another object of the invention is to provide a preparation which can remarkably reduce a concentration of fibrinogen in the blood, inhibit thrombus formation, and is useful for preventing or treating a diabetes-associated disease such as type II diabetes or metabolic syndrome.

It is still another object of the invention to provide a preparation which is capable of increasing a concentration of HDL-cholesterol in the blood effectively and a process for producing the preparation.

It is a further object of the invention to provide a preparation which has the activity even in a small dose effectively and a process for producing the preparation.

It is a still further object of the invention to provide a combined pharmaceutical preparation having a high safety and a process for producing the preparation.

### MEANS TO SOLVE THE PROBLEMS

The inventors of the present invention made intensive studies to achieve the above objects and finally found that a combined administration of at least a statin compound having a benzopyridine skeleton (e.g., pitavastatin) as a statin agent and a fibrate agent can (i) reduce a concentration of a free fatty acid and/or fibrinogen in the blood effectively or (ii) reduce a concentration of a free fatty acid and/or fibrinogen in the blood effectively and increase a concentration of HDL-cholesterol in the blood effectively. The present invention was accomplished based on the above findings.

That is, the preparation (or pharmaceutical composition) of the present invention is a preparation reducing a concentration of a free fatty acid and/or fibrinogen in the blood. The preparation contains a statin agent comprising at least a statin compound having a benzopyridine skeleton and a fibrate agent. The preparation may be a preparation for preventing or treating hyper-free fatty acidemia, metabolic syndrome, type II diabetes, type II diabetes complication, lipotoxicity, abnormal lipid metabolism, glucose intolerance, impaired insulin secretion, fatty liver, hypo-high density lipoproteinemia, or thrombosis, particularly may be a preparation for preventing or treating a disease caused by visceral fat or an excessive free fatty acid in the blood.

The fibrate agent may comprise at least one member selected from the group consisting of bezafibrate, clinofibrate, clofibrate, clofibratealuminum, fenofibrate, simfibrate, fenofibric acid, gemfibrozil, and a salt thereof.

The benzopyridine skeleton of the statin compound may have at least one substituent selected from the group consisting of a C₁₋₆alkyl group, a C₃₋₆cycloalkyl group, a C₆₋₁₀aryl group, and a halogenated C₆₋₁₀aryl group. Such a statin compound may comprise a benzopyridylethyl- or benzopyridylvinyl-substituted 3,5-dihydroxypentanoic acid or a derivative thereof, where the benzopyridine skeleton may have the above-mentioned substituent. The derivative may include a salt or lactone of the 3,5-dihydroxypentanoic acid (e.g., 3-hydroxy-δ-valerolactone). In the preparation, the statin agent may comprise at least pitavastatin. In the statin agent, the content of the statin compound having thebenzopyridineskeleton (e.g., pitavastatin) may be about 10 to 100% by weight. The weight ratio of the fibrate agent (e.g., fenofibrate) relative to 1 part by weight of the statin agent (e.g., pitavastatin) maybe, for example, about 1 to 500 parts by weight (for example, about 3 to 300 parts by weight). Incidentally, the preparation may be a liquid preparation. The preparation is usually a solid preparation containing a physiologically acceptable carrier.

Moreover, the present invention includes an agent (or a pharmaceutical agent) reducing a free fatty acid in the blood or an agent (or a pharmaceutical agent) reducing fibrinogen in the blood. The agent (or a pharmaceutical agent) comprises a combination of a statin agent comprising at least a statin compound having a benzopyridine skeleton and a fibrate agent as effective (or active) ingredients, and the proportion of the fibrate agent is 1 to 500 parts by weight (preferably, 3 to 300 parts by weight) relative to 1 part by weight of the statin agent.

The present invention can effectively reduce a free fatty acid content in the blood. Hyper-free fatty acidemia is a disease that a free fatty acid sustainedly (or continuously) fed from visceral fat or a free fatty acid retained in the blood increases. The hyper-free fatty acidemia causes influx and excessive take-up of a free fatty acid in a non-adipose tissue (for example, the liver, muscle, and pancreatic β-cell), thereby inducing a disease such as metabolic syndrome, type II diabetes, type II diabetes complication, lipotoxicity, abnormal lipid metabolism, glucose intolerance, impaired insulin secretion, fatty liver, or hypo-high density lipoproteinemia.

Incidentally, throughout this specification, the meaning of the term "pharmaceutical" (or "pharmaceutical preparation") includes a drug, a quasi drug, and the like. Further, the concept of the preparation in the present invention includes a pharmaceutical composition.

### EFFECTS OF THE INVENTION

According to the present invention, a combination of a specific statin agent and a fibrate agent can remarkably reduce a concentration of a free fatty acid and/or fibrinogen in the blood. In particular, such a combination can reduce an excessive free fatty acid in the blood and is useful for preventing or treating a disease or disorder (e.g., hyper-free fatty acidemia, metabolic syndrome, type II diabetes, type II diabetes complication, lipotoxicity, abnormal lipid metabolism, glucose intolerance, impaired insulin secretion, and fatty liver) caused by (or accompanied by) an excessive increase of the free fatty acid in the blood. Further, since the preparation of the present invention can reduce a concentration of fibrinogen in the blood and has a high antithrombogenicity, the preparation is useful for preventing or treating thrombosis caused by abnormalities of thrombocyte, coagulation-fibrinolytic system, or the like. In particular, the preparation of the present invention can reduce the concentration of fibrinogen in the blood inhibits a thrombus formation and is useful for preventing or treating a disease or disorder associated with diabetes (e.g., type II diabetes, type II diabetes complication, and metabolic syndrome), which easily induces thrombosis. Moreover, according to the present invention, the concentration of the free fatty acid and/or fibrinogen in the blood can be reduced, and a concentration of HDL-cholesterol in the blood can be raised effectively. In particular, the present invention is effective as an agent for reducing the free fatty acid in the blood, for example, is useful for preventing or treating a disease caused by (or accompanied by) visceral fat or an excessive free fatty acid in the blood or metabolic syndrome, whose risk factor is abnormal lipid metabolism caused by (or accompanied by) an excessive free fatty acid in the blood. Furthermore, the preparation of the present invention can effectively exhibit the activities even in a small dose. In addition, the preparation has a high safety.

### DETAILED DESCRIPTION OF THE INVENTION

The preparation (or pharmaceutical composition) of the present invention comprises a combination of a statin agent and a fibrate agent.

The statin agent is not particularly limited to a specific one as long as the agent is a component or agent which inhibits HMG-CoA reductase and has a lipid-lowering action (or lipid-reducing action), particularly, reduces cholesterols in the blood.

In particular, in the present invention, the above-mentioned statin agent comprises at least a statin compound having a benzopyridine skeleton (hereinafter, sometimes referred to as a "statin compound" simply). The statin compound may have a substituent on the benzopyridine skeleton. Incidentally, the meaning of the term "benzopyridine skeleton" includes a quinoline skeleton and an isoquinoline skeleton.

The statin compound may include 3,5-dihydroxypentanoic acid having a group such as a benzopyridylC₁₋₆alkyl group (such as quinolylethyl group) or a benzopyridylC₂₋₆alkenyl group (such as quinolylvinyl group) as a benzopyridine skeleton, or a derivative of the acid. Incidentally, the derivative may include a C₁₋₆alkyl ester (e.g., methyl ester) of the above-mentioned 3,5-dihydroxypentanoic acid, an amide of the above-mentioned 3,5-dihydroxypentanoic acid with a C₁₋₆alkylamine (e.g., methylamine), a salt or lactone of the above-mentioned 3,5-dihydroxypentanoic acid (specifically, a closed ring lactone form of the 3,5-dihydroxypentanoic acid, for example, 3-hydroxy-δ-valerolactone), and others. Among these derivatives, the salt [for example, a salt with an inorganic base such as an alkali metal (e.g., sodium and potassium), an alkaline earth metal (e.g., calcium and magnesium), or aluminum] or the lactone is preferred.

The substituent on the benzopyridine skeleton of the statin compound may include, for example, a C₁₋₆alkyl group such as methyl group; a C₃₋₆cycloalkyl group such as cyclopropyl or cyclobutyl group; a C₆₋₁₀aryl group such as phenyl group; and a halogenated C₆₋₁₀aryl group such as fluorophenyl, chlorophenyl, or bromophenyl group. The statin compound may have one of these substituents or a plurality of the same or different substituents. It is particularly preferable that the statin compound at least have a C₃₋₅cycloalkyl group and/or a fluoroC₆₋₁₀aryl group.

Among these statin compounds, the preferred one includes a benzopyridylethyl- or benzopyridylvinyl-substituted 3,5-dihydroxypentanoic acid in which the benzopyridine skeleton may have the above-mentioned substituent (that is, 7-benzopyridyl-3,5-dihydroxyheptanoic acid or 7-benzopyridyl-3,5-dihydroxy-6-heptenoic acid), particularly a quinolylethyl- or quinolylvinyl-substituted 3,5-dihydroxypentanoic acid (that is, 7-quinolyl-3,5-dihydroxyheptanoic acid or 7-quinolyl-3,5-dihydroxy-6-heptenoic acid), or a derivative thereof (e.g., a salt thereof with an inorganic base, and a lactone body thereof). It is particularly preferable that the statin compound have the substituent (for example, cyclopropyl group and/or fluorophenyl group (such as 4-fluorophenyl group)) on the benzopyridine skeleton.

Concrete examples of the statin compound include a 5-{2-[2-cyclopropyl-4-(p-fluorophenyl)-3-quinolyl]vinyl]-3,5-dihydroxypentanoic acid such as (3R,5S,6E)-7-[2-cyclopropyl-4-(4-fluorophenyl)-3-quinolyl]-3,5-dihydroxy-6-heptenoic acid, a salt thereof (for example, an alkali metal salt, an alkaline earth metal salt [a calcium salt such as pitavastatin (or pitavastatin calcium))] or a lactone body thereof, and others. In the present invention, the statin compound, particularly, the statin agent preferably comprises at least pitavastatin.

In the present invention, the statin agent is not particularly limited to a specific one as long as the agent comprises at least the above-mentioned specific statin compound. The statin agent may contain other statin compounds (active statin components), for example, pravastatin, simvastatin, fluvastatin, atorvastatin, lovastatin, cerivastatin, rosuvastatin, or salts thereof (for example, pravastatin sodium, fluvastatin sodium, and atorvastatin calcium hydrate). These other statin compounds may be used singly or in combination.

The proportion (content) of the statin compound havingabenzopyridineskeleton (forexample, pitavastatin) in the statin agent may be about 10 to 100% by weight (e.g., about 20 to 95% by weight), preferably about 25 to 100% by weight (e.g., about 30 to 90% by weight), and more preferably about 50 to 100% by weight (e.g., about 60 to 80% by weight).

The fibrate agent (or active fibrate component) is not particularly limited to a specific one as long as the agent is a component or agent having a lipid-lowering action, particularly, a component or agent for reducing a triglyceride level in the blood and/or a cholesterol level in the blood. For example, the agent may be a compound exhibiting a lipid-lowering action by inhibiting triglyceride synthesis or secretion in the liver, activating lipoprotein lipase, and others. Examples of the fibrate agent may include bezafibrate, clinofibrate, clofibrate, clofibrate aluminum, fenofibrate, simfibrate, fenofibric acid, gemfibrozil, or salts thereof. These fibrate agents may be used singly or in combination.

The preferred fibrate agent (or active fibrate component) includes fenofibrate, bezafibrate, and salts thereof (for example, a physiologically or pharmaceutically acceptable salt), particularly fenofibrate.

The above-mentioned salt of the fibrate agent and the salt of the statin agent (e.g., a physiologically or pharmaceutically acceptable salt) are not limited to the salts mentioned above and may include, for example, a salt with an inorganic or organic base, a salt with an inorganic or organic acid, and a salt with a neutral, basic, or acidic amino acid. The inorganic base may include, for example, an alkali metal such as sodium or potassium, an alkaline earth metal such as calcium or magnesium, aluminum, and ammonium. The organic base may include, for example, an alkylamine such as trimethylamine or triethylamine; a heterocyclic amine such as pyridine or picoline; an alkanolamine such as ethanolamine, diethanolamine, or triethanolamine; a cycloalkylamine such as dicyclohexylamine; and an alkylenediamine derivative such as N,N-dibenzylethylenediamine. The inorganic acid may include, for example, hydrochloric acid, hydrobromic acid, nitric acid, sulfuric acid, and phosphoric acid. The organic acid may include, for example, a monocarboxylic acid such as formic acid, acetic acid, or trifluoroacetic acid; a polycarboxylic acid such as fumaric acid, maleic acid, or oxalic acid; a hydroxycarboxylic acid such as tartaric acid, citric acid, succinic acid, or malic acid; and a sulfonic acid such as methanesulfonic acid, benzenesulfonic acid, or p-toluenesulfonic acid. The neutral amino acid may include, for example, glycine, valine, and leucine; the basic amino acid may include, for example, arginine, lysine, and ornithine; the acidic amino acid may include, for example, aspartic acid and glutamic acid.

Incidentally, the above-mentioned statin agent and fibrate agent also include derivatives of respective active compounds (e.g., an ester, a salt hydrate, and a hydrate) or prodrugs thereof. The statin agent or fibrate agent may be an optically active body or a racemic body.

The weight ratio of the above-mentioned specific statin agent (for example, pitavastatin) relative to the fibrate agent (for example, fenofibrate) [the former/the latter] in the preparation may be selected from the range of about 0.01/99.99 to 99/1 (e.g., about 0.1/99.9 to 90/10). The ratio of the both agents [the former/the latter] may be about 0.2/99.8 to 50/50, preferably about 0.3/99.7 to 30/70, and more preferably about 0.5/99.5 to 10/90.

Further, regarding the statin agent (or the statin compound (e.g., pitavastatin)) in a preparation containing the statin agent and the fibrate agent (e.g., fenofibrate) in a preparation containing the fibrate agent, the proportion of the fibrate agent relative to 1 part by weight of the statin agent (or statin compound (e.g., pitavastatin)) may usually be selected from the range of about 1 to 500 parts by weight, for example, about 3 to 300 parts by weight, preferably about 5 to 250 parts by weight (e.g., about 10 to 250 parts by weight), and more preferably about 15 to 200 parts by weight (e.g., about 20 to 150 parts by weight) or may be about 3 to 120 parts by weight, preferably about 5 to 100 parts by weight, and more preferably about 7 to 80 parts by weight, provided that, in the case of a combination of pitavastatin and fenofibrate, the proportion of 1 part by weight of fenofibrate relative to 1 part by weight of pitavastatin is usually excluded.

The preparation of the present invention may contain other agents for hyperlipidemia (for example, nicotinic acid and a derivative thereof (such as nicomol or niceritrol); an ion exchange agent; and probucol), an antianginal agent, a β-blocker, a Ca antagonist, an antiarrhythmic agent, a diuretic agent, a depressor (a sympathetic blocking agent such as a central α₂ agonist, a peripheral sympathetic blocking agent, an autonomic ganglionic blocking agent, an α-blocker, or the β blocker; a vasodilator; the above-mentioned Ca antagonist; an ACE inhibitor; and an angiotensin II receptor antagonist), a vasopressor, an agent for diabetes, an antiphlogistic agent, a vitamin compound (e.g., vitamin A, vitamin B, vitamin C, vitamin D, and vitamin E), an amino acid (e.g., cysteine), and others.

The form of the preparation of the present invention is not particularly limited to a specific one and may be a solid preparation (for example, powdered preparations, particles, spherical or spheroidal pills, pills, tablets, capsules, and suppositories), a semisolid preparation (for example, creams, ointments, and gels), aliquid preparation (for example, solutions, suspensions, emulsions, gumdrop-like preparations, syrup, elixir, lotions, and injectable solutions (or injections)), and others. Incidentally, the capsules may be a capsule having a liquid filled therein or a capsule having a solid preparation (such as granules) filled therein. Moreover, the preparation may be a lyophilized preparation. Further, an agent contained in the preparation of the present invention may be released at a controlled rate, that is, the preparation of the present invention may be a sustained release preparation or a rapid-release preparation.

Further, the preparation may be a preparation for oral administration or a preparation for parenteral administration. Furthermore, the preparation may be a preparation for topical administration or application (for example, solutions, suspensions, and poultices). The preparation of the present invention is practically a solid preparation (particularly, a preparation for oral administration).

The preparation usually contains a physiologically acceptable carrier. The carrier may be selected, depending on the dosage form, the route of administration, the application, and others, for example, from components listed in the Japanese Pharmacopoeia and "Encyclopedia of Pharmaceutical Excipients 2000 (Iyakuhin Tenkabutsu Jiten 2000)" (Yakuji Nippo Ltd., the second edition, issued on March 25, 2002). For example, at least one carrier selected from the group consisting of an excipient, a binder, and a disintegrant is practically used as a carrier for the solid preparation. An additive such as a lipid may also be used.

The excipient may include a saccharide or a sugar alcohol such as lactose, white soft sugar or refined sugar, glucose, sucrose, mannitol, or sorbitol; a starch such as a corn starch; a polysaccharide such as a crystalline cellulose (including a microcrystalline cellulose); silicon dioxide or a silicate such as a light silicic anhydride or a synthetic aluminum silicate; and others. The binder may include a water-soluble starch such as a pregelatinized starch or a partially pregelatinized starch; a polysaccharide such as agar, gum acacia (or gum arabic), dextrin, sodium alginate, a tragacanth gum, a xanthan gum, a hyaluronic acid, or a sodium chondroitin sulfate; a synthetic polymer such as a polyvinylpyrrolidone, a polyvinyl alcohol, a carboxyvinyl polymer, a polyacrylic polymer, a polylactic acid, or a polyethylene glycol; a cellulose ether such as a methyl cellulose, an ethyl cellulose, a carboxymethyl cellulose, a carboxymethyl cellulose sodium, a hydroxyethyl cellulose, a hydroxypropyl cellulose, or a hydroxypropylmethyl cellulose; and others. The disintegrant may include calcium carbonate, a carboxymethyl cellulose or a salt thereof (e.g., a carmellose, a carmellose sodium, and a carmellose calcium), a polyvinylpyrrolidone (e.g., a polyvinylpyrrolidone and a crosslinked polyvinylpyrrolidone (crosslinked povidone)), a low-substituted hydroxypropyl cellulose, and others. These carriers maybe used singly or in combination.

Examples of an oil-based carrier for the liquid preparation may include an oil derived fromplants or animals (e.g., an oil derived from vegetables such as a jojoba oil, an olive oil, a palm oil, or a cotton seed oil; and an oil derived from animals such as squalene), a mineral oil (e.g., a liquid petrolatum and a silicone oil), and others. An aqueous carrier may include water (e.g., a purified water or a sterile water, a distilled water for injection), a physiological saline, a Ringer's solution, a glucose solution, a water-soluble organic solvent [for example, a lower aliphatic alcohol such as ethanol or isopropanol; a (poly) alkylene glycol (e.g., ethylene glycol, diethylene glycol, and a polyethylene glycol); and glycerin], dimethyl isosorbide, dimethylacetamide, and others. Moreover, the carrier for the semisolid preparation may be selected from the carrier for the solid preparation and/or that for the liquid preparation. Further, the carrier for the semisolid preparation may also contain a lipid.

The lipid may include a wax (e.g., a bees wax, a carnauba wax, a lanolin, a paraffin, and a petrolatum), a higher (or long chain) fatty acid ester [e.g., an alkyl ester of a saturated or unsaturated fatty acid, and an ester of a fatty acid with a polyhydric (polyvalent) alcohol (such as a polyC₂₋₄alkylene glycol, glycerin, or a polyglycerin) (e.g., a glyceride)], a hardened (or hydrogenated) oil, a higher alcohol (e.g., a saturated aliphatic alcohol such as stearyl alcohol and an unsaturated aliphatic alcohol such as oleyl alcohol), a higher fatty acid (e.g., stearic acid and oleic acid), a metallic soap (e.g., a metal salt of a fatty acid, such as a sodium salt of palm oil fatty acid or calcium stearate), and others.

The additive may include, for example, a lubricant (e.g., a talc, magnesium stearate, and a polyethylene glycol 6000), a disintegrant aid, an antioxidation agent or an antioxidant, an emulsifier (e.g., a variety of surfactants such as a nonionic surfactant), a dispersing agent, a suspending agent, a dissolution aid, a thickener (e.g., a water-soluble polymer such as a carboxyvinyl polymer, a polyvinyl alcohol, a carrageenan, or a gelatin; and a cellulose ether such as a carboxymethyl cellulose), a pH adjusting agent or a buffer (e.g., a citric acid-sodium citratebuffer), an antiseptic agent or a preservative (e.g., a paraben such as methyl paraben or butyl paraben), a fungicide or antibacterial agent (e.g., a benzoic acid compound such as sodium benzoate), an antistatic agent, a corrigent or a masking agent (e.g., a sweetening agent), a coloring agent (e.g., a dye and a pigment such as colcothar), a deodorant or a perfume (e.g., an aromatic substance), an algefacient, an antifoaming agent, an isotonizing agent, and a soothing agent. These additives may be used singly or in combination.

The preparation of the present invention comprising a combination of a specific statin agent and a fibrate agent can synergistically reduce a concentration of a free fatty acid in the blood compared with the use of the specific statin agent alone and the use of the fibrate agent alone. Further, the preparation can reduce a concentration of fibrinogen in the blood, thereby inhibiting thrombus formation followed by fibrin coagulation and/or thrombocyte cohesion. In particular, a disease associated with diabetes (e.g., type II diabetes, type II diabetes complication, and metabolic syndrome) facilitates adhesion or cohesion of thrombocyte due to abnormal thrombocyte, abnormal coagulation-fibrinolytic system, and others, whereby thrombus is easily formed. However, even in the state where thrombus is easily formed, the above-mentioned combination ensures reduction of a concentration of fibrinogen in the blood and realizes a high antithrombogenicity. Therefore, the combination is effective for preventing or treating a disease associated with the diabetes. Moreover, the above-mentioned combination can effectively raise a concentration of HDL-cholesterol in the blood.

Further, the preparation (or pharmaceutical composition) of the present invention has a high safety. For example, the incidence of an adverse event in the use of a statin agent other than the specific statin agent in a clinical test is about 9%, while the specific statin compound (e.g., pitavastatin) and the fibrate agent (e.g., fenofibrate) have a high safety and the incidence of an adverse event in a clinical test can be reduced to about 5 to 6%. The proportions of the statin agent and the fibrate agent in the preparation may be selected depending on the subject to be applied or administered, the age and body weight, the condition, the number of administrations, the method (or route) of administration, and others. The content of the specific statin compound (or statin agent) and the fibrate agent mainly depends on a dose. For example, the total content of the specific statin compound and the fibrate agent in the preparation may be, for example, about 0.01 to 99% by weight, preferably about 0.1 to 95% by weight, and more preferably about 1 to 80% by weight (e.g., about 5 to 50% by weight) in terms of a solid content. The total content of the specific statin compound (or statin agent) and the fibrate agent in the preparation is usually about 5 to 70% by weight and preferably about 10 to 50% by weight.

Incidentally, the present invention also includes a kit comprising a preparation containing a statin agent and a preparation containing a fibrate agent in combination.

The preparation of the present invention can be produced by a conventional production process using the statin agent, the fibrate agent, and the carrier (for example, a physiologically acceptable carrier). For example, the solid preparation may be, for example, produced by using a carrier together with effective ingredients (the specific statin compound (or statin agent) such as pitavastatin and the fibrate agent). For example, the granules may be prepared by granulating the effective ingredient and the carrier component through extrusion granulation, spray granulation, or other means, and if necessary regulating the size of the resulting granule. The tablets may be produced by mixing the granulated matter and the carrier and/or additive if necessary, and compression-molding the resultant. Moreover, if necessary, the granules or tablets may be coated by per se known methods for masking the taste or imparting gastro-soluble property, enteric property or durability thereto. The capsules maybe prepared by filling granules or liquid preparation in a capsule.

The liquid preparation may be prepared by mixing (e.g., dissolving, suspending, and emulsifying) the effective ingredients and the liquid carrier (e.g., an aqueous carrier such as a purified water, and an oil-based carrier), and if necessary, the carrier for the solid or semisolid preparation, the additive (e.g., an emulsifier, a dispersing agent, a suspending agent, an isotonizing agent, a dissolution aid, a preservative, a corrigent, and a pH adjusting agent or a buffer), and if necessary, the liquid preparation is sterilized.

The semisolid preparation may be prepared by mixing or kneading the effective ingredients and the carrier for the semisolid preparation (and if necessary, the additive), if necessary under heating.

The amount to be administered (or dose) of the preparation of the present invention and the administration schedule thereof may be selected in accordance with the route of administration, the degree of the disease of the patient (or subject), and the age, sex, and body weight of the patient (or subject), and usually the pharmacological activity of the preparation can effectively be exhibited even in a small dose. For example, the dose of the statin compound (or statin agent) such as pitavastatin may be about 0.1 to 10 mg, preferably about 0.5 to 7 mg, and about more preferably 1 to 5 mg to an adult human being per day. The dose of the fibrate agent maybe about 1 to 500 mg, preferably about 5 to 300 mg (e.g., about 10 to 250 mg), and more preferably about 30 to 200 mg (e.g., about 50 to 150 mg) to an adult human being per day.

Incidentally, the form of administration of the kit is not particularly limited to a specific one. For example, a preparation containing the statin compound and a preparation containing the fibrate agent may be administered at the same time (simultaneously) or separately. In the separate administration, these preparations may be administered separately to a subject leaving a period of time in between. While one preparation precedently administered is maintaining the effects of the active component contained therein, the other preparation is usually administered. When a preparation containing the statin compound and a preparation containing the fibrate agent are administered separately, it is preferable that the both preparations be administered simultaneously or that one preparation be administered immediately after administration of the other preparation. Moreover, a preparation containing the statin compound and a preparation containing the fibrate agent may be administered in the form of a mixture by mixing these preparations (and a diluent or the like, if necessary) before (for example, immediately before) administration.

### INDUSTRIAL APPLICABILITY

The preparation of the present invention can reduce a free fatty acid level in the blood and is useful for a preventive (prophylactic) or treating (therapeutic) agent for a disease or disorder caused by (or accompanied by) an excessive increase in the free fatty acid level in the blood, e.g., hyper-free fatty acidemia, metabolic syndrome, type II diabetes, type II diabetes complication, lipotoxicity, abnormal lipid metabolism, glucose intolerance, impaired insulin secretion, or fatty liver. Moreover, since the preparation of the present invention can reduce a concentration of fibrinogen in the blood, the preparation inhibits thrombus formation and is useful for an antithrombotic agent. Further, the preparation reduces a concentration of a free fatty acid in the blood and is useful for a preventive (prophylactic) or treating (therapeutic) agent for hypo-high density lipoproteinemia.

### EXAMPLES

The following examples are intended to describe this invention in further detail and should by no means be interpreted as defining the scope of the invention.

### Example 1 (Test Example)

Pitavastatin (PIT) was added to a food and was given to normal rabbits (rabbits with normal blood lipid levels) in a dose of 0.5 mg/kg/day for 4 weeks (PIT administration group). Fenofibrate (FEN) was added to a food and was given to normal rabbits in a dose of 30 mg/kg/day for 4 weeks (FEN administration group). PIT and FEN were added to a food and was given to normal rabbits for 4 weeks (combination administration group). Incidentally, in the combination administration group, the dose of PIT was 0.5 mg/kg/day and that of FEN was 30 mg/kg/day.

After the final administration of the agents, these rabbits were fasted for one day, and each blood was collected from the auricular vein and treated with ethylenediamine tetraacetic acid (EDTA) for preparing a plasma. The total cholesterol (TC) and the triglyceride (TG) concentration in the plasma were measured in accordance with the enzymatic method by using an assay kit for enzymatic method (manufactured by Wako Pure Chemical Industries, Ltd.). In addition, part of the plasma was ultracentrifuged to separate into the following lipoprotein fractions: low-density lipoprotein (LDL), very low-density lipoprotein (VLDL), and intermediate-density lipoprotein (IDL). The cholesterol and TG in each lipoprotein were measured. After collection of blood, each rabbit was slaughtered. The liver was extracted and homogenized to measure the cholesterol content in the liver in accordance with the enzymatic method.

The results revealed that the combination administration group, in which both of PIT and FEN had been administered, showed remarkable reduction in the TC and TG levels, compared with the PIT administration group, in which PIT had been administered alone, and the FEN administration group, in which FEN had been administered alone. Moreover, the combination administration group exhibited further lower TC and TG values, compared with the decrease in the TC and TG levels predicted based on the PIT administration group and the FEN administration group.

### Example 2 (Test Example)

Pitavastatin (PIT) was orally administered to normal guinea pigs (guinea pigs with normal blood lipid levels) in a dose of 1 mg/kg/day for 2 weeks (PIT administration group). Fenofibrate (FEN) was orally administered to normal guinea pigs in a dose of 30 mg/kg/day for 2 weeks (FEN administration group). PIT and FEN were orally administered to normal guinea pigs for 2 weeks (combination administration group). Incidentally, in the combination administration group, the dose of PIT was 1 mg/kg/day and that of FEN was 30 mg/kg/day.

After the final administration of the agent, these guinea pigs were fasted for one day, and each blood was collected from the aorta abdominalis. Using the same manner as in Example 1, a plasma was prepared, the TC and TG values were measured, and the cholesterol content in the liver was measured.

The results revealed that the combination administration group, in which both of PIT and FEN had been administered, showed remarkable reduction in the TC and TG levels, compared with the PIT administration group, in which PIT had been administered alone, and the FEN administration group, in which FEN had been administered alone. Moreover, the combination administration group exhibited further lower TC and TG values, compared with the decrease in the TC and TG levels predicted based on the PIT administration group and the FEN administration group.

### Example 3 (Test Example)

Examination of decrease of lipid and others in blood by combinationuseoffenofibrate (FEN) andpitavastatin (PIT) in dog

### (1) Subject

Dogs [female and male beagles (YAKUKEN BEAGLE) (11 male dogs and 2 female dogs, 27 to 73 months old)] were used for the examination. Incidentally, each of the dogs was accommodated in a separate bracket cage in a breeding room under the following conditions: a room temperature of 23±3°C, a humidity of 50±10%, a light period of 8:00 to 20:00, and a dark period of 20:00 to 8:00. Moreover, the dog was given 300g of CD-5M (manufactured by CLEA Japan, Inc.) as a food once a day. Incidentally, the dog was freely allowed to have tap water.

### (2) Test substance and control substance

Fenofibrate (FEN) (20mg/kg) andpitavastatin (PIT) (2 mg/kg) were used as test substances.

### (3) Test

Fourhours (Day0-4hr) and 24 hours (DayO-24hr) after feeding on the day before the administration start day, blood was collected from antebrachial radial veins, and serum and plasma containing sodium citrate (a citric sodium aqueous solution having a concentration of 3.8% by weight) were obtained. Then using the serum, the total cholesterol (TC), the triglyceride (TG), the phospholipid (PL), and the free fatty acid (NEFA) were quantitatively determined by a Toshiba automatic analyzer (manufactured by Toshiba Medical Systems Corporation, TBA-120FR). Moreover, using the sodium citrate-containing plasma, the amount of fibrinogen was quantitatively determined in accordance with the thrombin time method.

Based on the quantitation results, 12 dogs which showed relatively stable value of the serum lipid at both the Day0-4hr and the Day0-24hr were selected, and every 4 dogs formed a group based on the TC value as a index (that is, three groups were formed). Thereafter, FEN alone in a dose of 20 mg/kg was orally administered to a first group of dogs once a day for continuous 7 days (FEN group); PIT alone in a dose of 2 mg/kg was orally administered to a second group of dogs once a day for continuous 7 days (PIT group); and FEN in a dose of 20 mg/kg and PIT in a dose of 2 mg/kg were orally administered to a third group of dogs once a day for continuous 7 days (combination group). Incidentally, feeding was performed immediately after every administration.

Blood was collected after 4 hours of the seventh administration (Day7-4hr). Using the same manner as in the case of the Day0-4hr, serum and sodium citrate-containing plasma were obtained, and each of the examinations was performed. Table 1 shows the list of each group.

[Table 1]

**Table 1**

| Group | Group name | Treatment | Number of dogs |
|---|---|---|---|
| 1 | FEN group | Oral administration of FEN 20 mg/kg for 7 days | 4 |
| 2 | PIT group | Oral administration of PIT 2 mg/kg for 7 days | 4 |
| 3 | Combination group | Oral administration of FEN 20 mg/kg and PIT 2 mg/kg for 7 days | 4 |

### (4) Statistic treatment of quantitation results

The quantitation results were shown as "an average value ± a standard error". The statistics analysis was performed as follows: each difference between the quantitative value at Day0-4hr and each at the blood-collected times after the administration was calculated with respect to every dog, and a difference between the average quantitative value of the FEN group or PIT group and that of the combination group was tested by using Student's t test (one-sided test) when homoscedasticity therebetween was identified by F test or by using Wilcoxon Rank Sum test (one-sided test) when homoscedasticity therebetween was not identified by F test. Incidentally, the obtained value from each test was regarded as a significant difference when the difference value was less than 5%.

### (5) Results

The results in the Day7-4hr are shown in Table 2. Moreover, Table 2 shows a relative index of a concentration of each component in the blood calculated with respect to each of the single administration groups and the combination group, and a product of the relative index of the FEN group and that of the PIT group. Incidentally, the relative index of each component was calculated based on the formula: (the average value of the concentration in the blood after the administration)/(the average value of the concentration in the blood before the administration).

[Table 2]

**Table 2**

| | Day7-4hr | | | |
|---|---|---|---|---|
| | FEN group | PIT group | Product of relative index of FEN group and that of PIT group | Combination group |
| ΔTC (mg/dL) Relative index | -21.4±3.9 0.827 | -25.0±3.6 0.797 | 0.659 | -50.4±12.2 0.595 |
| ΔTG (mg/dL) Relative index | -38.7±16.3 0.308 | -11.5±4.8 0.791 | 0.244 | -52.4±10.5 0.185 |
| ΔPL (mg/dL) Relative index | -71±11 0.773 | -61±9 0.795 | 0.615 | -126±35 0.586 |
| ΔNEFA (µEq/L) Relative index | -25±20 0.887 | -6±102 0.978 | 0.867 | -94±26 0.613 |
| ΔFibrinogen (mg/dL) | 6±22 | 11±11 | | -4±48 |

As apparent from Table 2, compared with the FEN group, the combination group showed significantly lower values in ΔTC, ΔNEFA, and Δfibrinogen. Incidentally, the significant difference of ΔTC and that of ΔNEFA were P<0.05 and P<0.05, respectively. Moreover, compared with the PIT group, the combination group showed significantly lower values in ΔTC and ΔTG. The significant difference of ΔTC and that of ΔTG were P<0.05 and P<0.01, respectively, compared with the PIT group. The lowering of the lipid level in the combination group was probably caused by an additive effect of FEN and PIT, while the lowering of ΔNEFA and Δfibrinogen was probably induced by a synergistic effect of FEN and PIT. Incidentally, it is known that the lowering of the TG level in the blood raises HDL-cholesterol level regardless of the species of animals (Progress in Medicine, Vol. 17, No.2, 1997. 2, pp. 291 to 296, and Arteriosclerosis, Thrombosis, and Vascular Biology, Vol. 15, No 11, November 1995, pp. 1819 to 1828). That is, the significant lowering of TG level in the above-mentioned Test Examples induces significant increase of the concentration of HDL-cholesterol in the blood.

Moreover, when the product of the relative index of the FEN group and that of the PIT group was calculated and compared with the relative index of the combination group based on Bulge formula (TAKAGI Keijiro et al.: Pharmacology, 1987, Nanzando Co., Ltd.), the product of the relative indices of the single administration groups was larger than the relative index of the combination group with respect to all components (that is, TC, TG, PL, and NEFA) as apparent from Table 2. It is clear that the combination administration has a synergistic effect.

Incidentally, as described in Examples of WO2006/011495 specification, when pitavastatin in a dose of 10 mg/kg and/or fenofibrate in a dose of 10 mg/kg are used, the product of the relative indices of the single administration groups is 0.650, while the relative index of the combination group is 0.627, with respect to TG. The difference therebetween is small. In contrast, from the results shown in Table 2, the relative index of the combination group is 0.185, and the product of the relative indices of the single administration groups is 0.244, with respect to TG. From the comparison, it is clear that reducing the amount of the specific statin agent is remarkably effective in lowering the concentration of TG in the blood.

## Claims

1. Apreparation reducing a concentration of a free fatty acid and/or fibrinogen in blood, the preparation comprising:
a statin agent comprising at least a statin compound having a benzopyridine skeleton and
a fibrate agent.

2. A preparation according to claim 1, which is a preparation for preventing or treating hyper-free fatty acidemia, metabolic syndrome, type II diabetes, type II diabetes complication, lipotoxicity, abnormal lipid metabolism, glucose intolerance, impaired insulin secretion, fatty liver, hypo-high density lipoproteinemia, or thrombosis.

3. A preparation according to claim 1, which is a preparation for preventing or treating a disease caused by visceral fat or an excessive free fatty acid in blood.

4. A preparation according to claim 1, wherein the fibrate agent comprises at least one member selected from the group consisting of bezafibrate, clinofibrate, clofibrate, clofibrate aluminum, fenofibrate, simfibrate, fenofibric acid, gemfibrozil, and a salt thereof.

5. A preparation according to claim 1, wherein the statin compound comprises a benzopyridylethyl- or benzopyridylvinyl-substituted 3,5-dihydroxypentanoic acid or a derivative thereof,
wherein a benzopyridine skeleton of the benzopyridylethyl or benzopyridylvinyl group may have a substituent selected from the group consisting of a C₁₋₆alkyl group, a C₃₋₆cycloalkyl group, a C₆₋₁₀aryl group, and a halogenated C₆₋₁₀aryl group, and
the derivative is a salt or lactone of the 3,5-dihydroxypentanoic acid.

6. A preparation according to claim 1, wherein the statin agent comprises at least pitavastatin, and the proportion of the fibrate agent is 1 to 500 parts by weight relative to 1 part by weight of the statin agent.

7. A preparation according to claim 1, wherein the proportion of the fibrate agent is 3 to 300 parts by weight relative to 1 part by weight of the statin agent.

8. A preparation according to claim 1, which is a solid preparation containing a physiologically acceptable carrier.

9. An agent for reducing a free fatty acid in blood, comprising a combination of
a statin agent comprising at least a statin compound having a benzopyridine skeleton and
a fibrate agent
as effective ingredients,
wherein the proportion of the fibrate agent is 3 to 300 parts by weight relative to 1 part by weight of the statin agent.

10. An agent for reducing fibrinogen in blood, comprising a combination of
a statin agent comprising at least a statin compound having a benzopyridine skeleton and
a fibrate agent as effective ingredients,
wherein the proportion of the fibrate agent is 3 to 300 parts by weight relative to 1 part by weight of the statin agent.

11. A method for preventing or treating hyper-free fatty acidemia, metabolic syndrome, type II diabetes, type II diabetes complication, lipotoxicity, abnormal lipid metabolism, glucose intolerance, impaired insulin secretion, fatty liver, hypo-high density lipoproteinemia, or thrombosis by using a preparation recited in claim 1.

12. Use of a preparation recited in claim 1 for preparing a preparation capable of preventing or treating hyper-free fatty acidemia, metabolic syndrome, type II diabetes, type II diabetes complication, lipotoxicity, abnormal lipid metabolism, glucose intolerance, impaired insulin secretion, fatty liver, hypo-high density lipoproteinemia, or thrombosis.
